# EUROPEAN PATENT SPECIFICATION

(11) **EP 2 066 692 B1**
(45) Date of publication and mention of the grant of the patent: **11.01.2012**
(21) Application number: 07803283.6
(22) Date of filing: 06.09.2007
(51) Int. Cl.: C07K 14/47, G01N 33/68

(54) **MEANS AND METHODS FOR THE PRODUCTION OF AMYLOID OLIGOMERS**
MITTEL UND VERFAHREN ZUR HERSTELLUNG VON AMYLOIDOLIGOMEREN
MOYENS ET PROCEDES POUR LA PRODUCTION D'OLIGOMERES AMYLOIDES

(30) Priority: 08.09.2006 EP 06120346; 08.09.2006 US 843076 P; 04.05.2007 US 927681 P
(43) Date of publication of application: 10.06.2009
(73) Proprietor: VIB, vzw, 9052 Gent (BE); VRIJE UNIVERSITEIT BRUSSEL, 1050 Brussel (BE); Katholieke Universiteit Leuven, K.U. Leuven R&D, 3000 Leuven (BE)
(72) Inventor: ROUSSEAU, Frederic, 1702 Groot-Bijgaarden (BE); SCHYMKOWITZ, Joost, 3391 Meensel-Kiezegem (BE); DA ROCHA MARTINS, Ivo, 4440-633 VALONGO (PT); DE STROOPER, Bart, 3000 Leuven (BE)
(86) International application number: PCT/EP2007/059327
(87) International publication number: WO 2008/028939

(56) References cited:
- WO-A-2004/007545
- TERZI E ET AL: "Self-association of beta-amyloid peptide (1-40) in solution and binding to lipid membranes" JOURNAL OF MOLECULAR BIOLOGY, LONDON, GB, vol. 252, no. 5, 1995, pages 633-642, XP002254797 ISSN: 0022-2836
- BOKVIST M ET AL: "Two Types of Alzheimer's beta-Amyloid (1-40) Peptide Membrane Interactions: Aggregation Preventing Transmembrane Anchoring Versus Accelerated Surface Fibril Formation" JOURNAL OF MOLECULAR BIOLOGY, LONDON, GB, vol. 335, no. 4, 23 January 2004 (2004-01-23), pages 1039-1049, XP004481649 ISSN: 0022-2836
- MCLAURIN J ET AL: "Membrane disruption by Alzheimer beta-amyloid peptides mediated through specific binding to either phospholipids or gangliosides. Implications for neurotoxicity." THE JOURNAL OF BIOLOGICAL CHEMISTRY 25 OCT 1996, vol. 271, no. 43, 25 October 1996 (1996-10-25), pages 26482-26489, XP002458373 ISSN: 0021-9258
- MCLAURIN J ET AL: "Review: Modulating factors in amyloid-beta fibril formation" JOURNAL OF STRUCTURAL BIOLOGY, ORLANDO, US, vol. 130, no. 2-3, June 2000 (2000-06), pages 259-270, XP002280181 ISSN: 1047-8477
- YIP C M ET AL: "Amyloid-beta peptide assembly: A critical step in fibrillogenesis and membrane disruption" BIOPHYSICAL JOURNAL, NEW YORK, US, US, vol. 80, no. 3, March 2001 (2001-03), pages 1359-1371, XP002254798 ISSN: 0006-3495
- JARRETT JOSEPH T ET AL: "Seeding "one-dimensional crystallization" of amyloid: A pathogenic mechanism in Alzheimer's disease and scrapie?" CELL, vol. 73, no. 6, 1993, pages 1055-1058, XP002458374 ISSN: 0092-8674
- KAYED R ET AL: "Common structure of soluble amyloid oligomers implies common mechanism of pathogenesis" SCIENCE, AMERICAN ASSOCIATION FOR THE ADVANCEMENT OF SCIENCE,, US, vol. 300, no. 5618, 18 April 2003 (2003-04-18), pages 486-489, XP002379307 ISSN: 0036-8075 cited in the application

## Description

### Field of the invention

The present invention relates to the field of amyloid disorders, more particularly to the field of diseases where protein misfolding leads to the generation of insoluble amyloid fibers in tissues and organs. The invention provides methods for the production of soluble, toxic amyloid oligomers. The invention further provides assays using said amyloid oligomers to screen for molecules that interfere with the toxicity of said oligomers.

### Background of the invention

The biological function of cells depends on the correct folding of a network of thousands of proteins. The information required to fold a protein into a functional, specific three-dimensional structure is contained in its amino acid sequence. In general, proteins fold properly into their native conformation and, if they do not, the misfolding is corrected by chaperone proteins. In amyloidogenic diseases, however, misfolding of a protein results in its aggregation and accumulation as protein deposits (amyloid fibers) in diverse tissues. Amyloid fibers (or fibrils) appear in electron micrographs as 100 angstrom diameter twisted rods composed of a cross-beta-sheet structure that selectively bind the dye Congo red and the environment-dependent fluorophore thioflavin T. Among the best known amyloidogenic diseases are Alzheimer's disease, Parkinson's disease, Huntington's disease and transmissible spongiform encephalopathies (TSEs). Although the causal proteins involved in these diseases do not share sequence or structural identity, all of them can adopt at least two different conformations without requiring changes in their amino acid sequence. The misfolded form of the protein usually contains stacks of β sheets organized in a polymeric arrangement known as a 'cross-β' structure. Because β sheets can be stabilized by intermolecular interactions, misfolded proteins have a high tendency to form amyloid oligomers and larger amyloid fibers. Compelling data from biochemical, genetic and several neuropathological studies support the involvement of protein misfolding and aggregation in the pathology of amyloid disorders. Indeed abnormal aggregates are usually present in the tissues with most damage and accumulation of these deposits in diverse organs is the endpoint in most amyloidogenic diseases. Mutations in the gene encoding the misfolded protein produce inherited forms of the disease, which usually have an earlier onset and a more severe phenotype than the sporadic forms. Central unresolved problems in understanding amyloid disorders are the nature and the formation of the molecular entities causing these diseases. Alzheimer's disease is an example of an amyloid disorder associated with the aggregation of amyloid-beta-peptide (Abeta-peptide) in amyloid plaques (which consist of amyloid fibers). The aggregation process starts with monomers (a single peptide unit each), proceeds to dimers (pairs), to trimers (trios), to oligomers (many units), to tiny transient structures known as protofibrils, to larger stable fibrils, and ends with highly compacted admixtures of fibrils and smaller aggregates (amyloid plaques). Neurotoxicity, however, is believed to be caused upstream in the Aβ-peptide aggregation process, by soluble amyloid oligomers, and not by the amyloid fibers themselves. WO2006004824 describes a specific, soluble 56 kDa Abeta oligomer as responsible for memory impairment prior to neuritic plaque formation. GM1 ganglioside-bound amyloid beta-protein, found in brains exhibiting early pathological changes of Alzheimer's disease, has been suggested to accelerate amyloid fibril formation by acting as a seed (Kakio A et al (2001) J Biol Chem 276(27):24985-90). Amyloid fibers which are the end product of the aggregation process are considered to be biologically inert. It is generally accepted in the art that these amyloid fibers are extremely stable under conditions that denature typical globular proteins and that the aggregation reaction cannot be reversed, i.e. amyloid fibers cannot generate soluble amyloid oligomers. Soluble amyloid oligomers, such as the toxic soluble amyloid beta oligomers, are valuable assay products but are difficult to obtain and cumbersome techniques are required for their purification such as the preparation of brain extracts followed by fractionation and immuno-affinity purification. We have surprisingly found a process to produce soluble amyloid oligomers starting form biologically inert amyloid fibrils. Our methodology uses lipids to disassemble amyloid fibers into soluble amyloid oligomers. Starting from amyloid beta fibers, the amyloid beta oligomers formed by our process are immunoreactive with the A11 epitope, indicative for toxic oligomerisation. We could also demonstrate lipid-induced neurotoxicity with other disease-associated amyloid and synthetic amyloid peptides demonstrating that lipid-induced cell toxicity by amyloid fiber disassembly is a generic property of amyloid fibers. The toxic amyloid oligomers can be conveniently used in *in vitro* and *in vivo* assays to screen for molecules that can interfere with their formation respectively toxicity.

### Figures

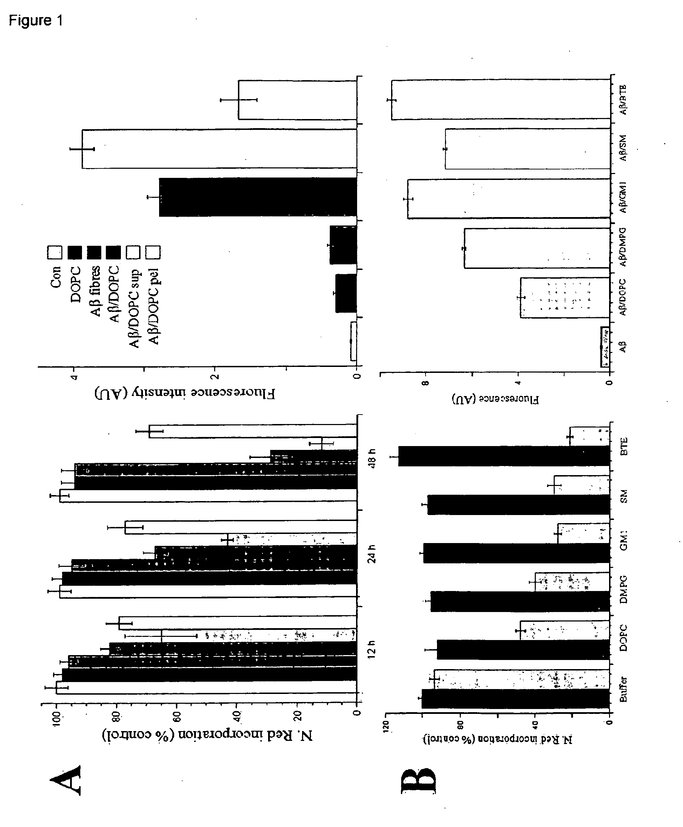

Figure 1: Lipids induce toxicity in mature Aβ42 amyloid fibers. (A) Left - Neutral red incorporation by neurons treated with respectively buffer, dioleyl phosphatidylcholine (DOPC) liposomes, Ab42 amyloid fibers, fiber/lipid mixtures, soluble fraction and resuspended insoluble fraction of fiber/lipid mixtures is shown for 12, 24 and 48h incubation periods. Right - annexin V fluorescence intensity at the 24h timepoint. (B) Left - Neutral red incorporation by neurons treated with liposomes (black) or the soluble fraction of amyloid/lipid mixtures (grey) is shown for DOPC, dioleyl phosphatidylglycerol (DMPG), ganglioside GM1 (GM1), sphingomyelin (SM) and brain total extract (BTE). Right - annexin V fluorescence intensity for the same samples.

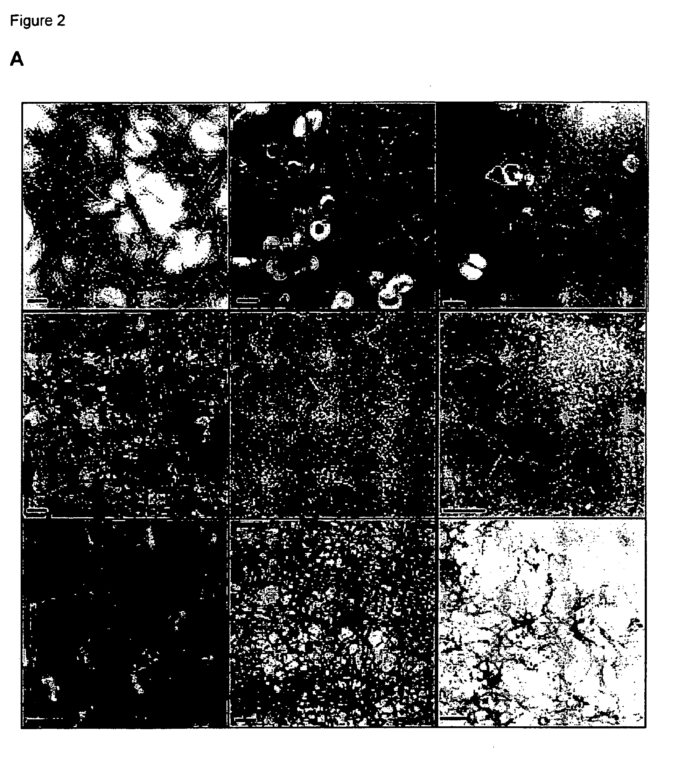

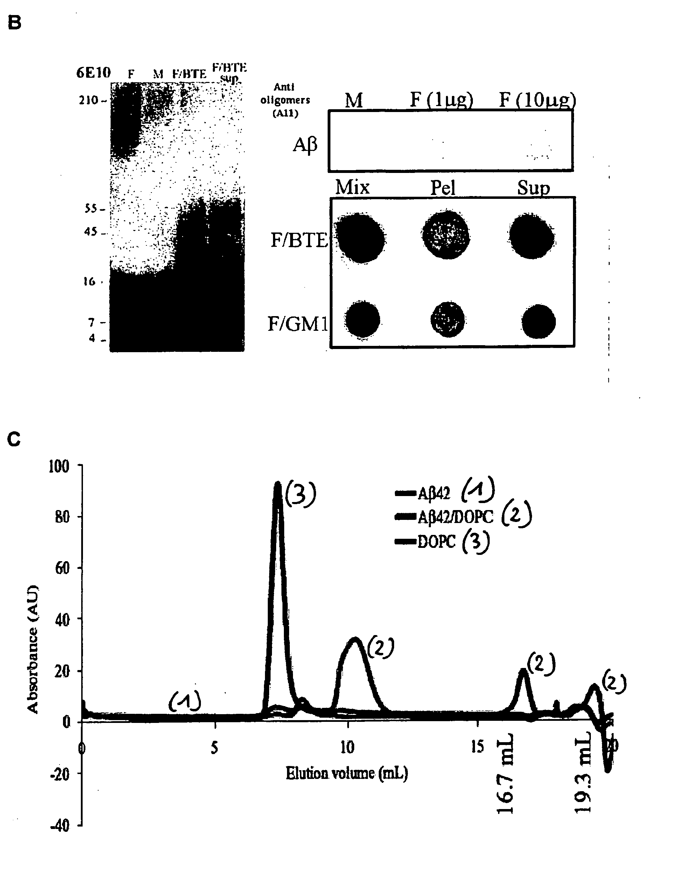

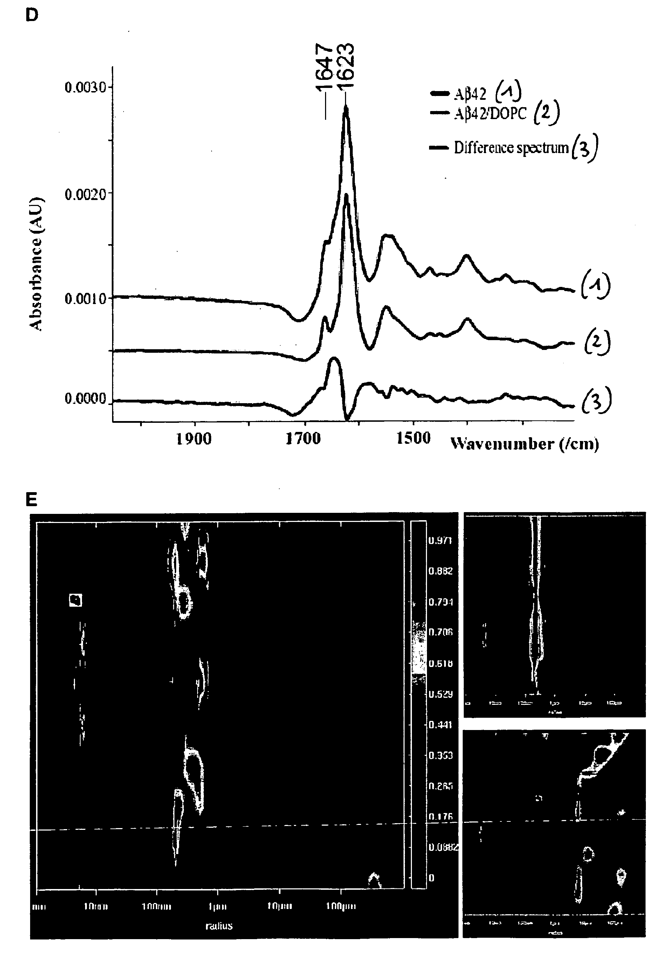

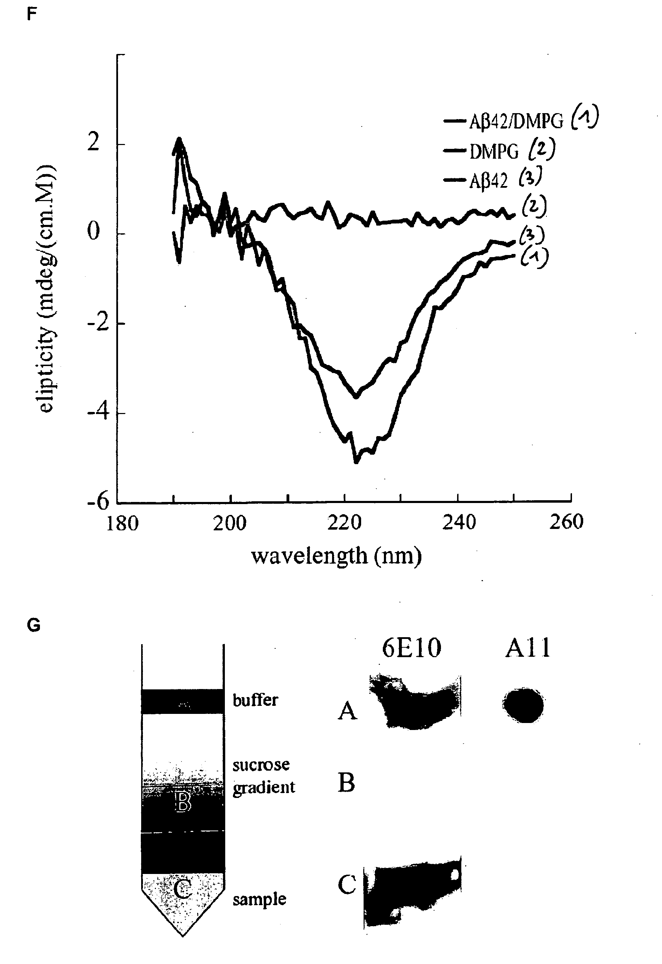

Figure 2: Lipids induce disassembly of mature Aβ42 amyloid fibers into soluble oligomers. (A) Electron microscopy pictures of mature Ab42 amyloid fibers alone (top left image) or mixed with DOPC liposomes. Notice the close interaction of liposomes and fibrils (top middle and right images). After centrifugation of a pellet that contains strongly intertwined lipids and amyloid fiber material (middle left image), and a soluble fraction that contains small oligomeric fragments (middle and right images) is generated. Similar effects are observed with other lipids, images display Aβ42 amyloid fibers mixed with liposomes containing BTE (bottom left), DMPG (bottom middle) and cholesterol (bottom right). (B) SDS-PAGE of mature Aβ42 amyloid fibers (F), Aβ42 monomers (M), amyloid/BTE lipid mixture (F/BTE) and the soluble fraction thereof (F/BTE sup), immunostained with 6E10 antibody. Dot blot of Aβ42 amyloid fibers alone (Aβ) or fibers mixed with BTE or with GM1 and the respective supernatant and pellet fractions probed with the oligomer specific antibody A11 . (C) Superimposed size Exclusion chromatography profile of DOPC liposomes, Aβ42 amyloid fibers and the soluble fraction of the amyloid/DOPC mixture. Liposomes elute in the void volume (first peak at 7.6 mL). The soluble fraction of the amyloid/lipid mixture reveals several peaks consistent with the presence of smaller oligomers. (D) Fourier Transform Infrared spectroscopy (FTIR) of mature Aβ42 fibers, fiber/lipid mixtures (BTE). Although a strong cross-beta signal remains, the difference spectrum reveals a small amount of random coil, consistent with partial fiber disassembly. (E) Dynamic Light Scattering (DLS) of mature Aβ42 fibers, liposomes and fiber/lipid mixtures (DOPC), revealing a marked increase in oligomers with sizes ranging between 10 and 100 nm. (F) Far UV circular dichroism (CD) spectroscopy of Aβ42 amyloid fibers in isolation and in the presence of liposomes containing a range of lipids displays a change in the intensity of the spectrum while the shape of the spectrum is constant, consistent with a change in the amount of soluble material that is present in the sample. A typical spectrum for DMPG is shown. (G) 'Floating assay' by ultracentrifugation reveals Alzheimer Aβ42 oligomers in association with liposomes in amyloid/lipid mixtures. After centrifugation for 1h at 150.000g, liposomes are in the top fraction whereas protein aggregates are expected in the pellet. As 6E10 immunostaining indicates, some Aβ42 material is indeed removed to the pellet, but a significant amount is transported to the top fraction in association with the lipids. This fraction is equally recognised by the A11 mAb, consistent with fiber disassembly, whereas the pellet fraction is not recognised by A11 and most likely contains intact fibers.

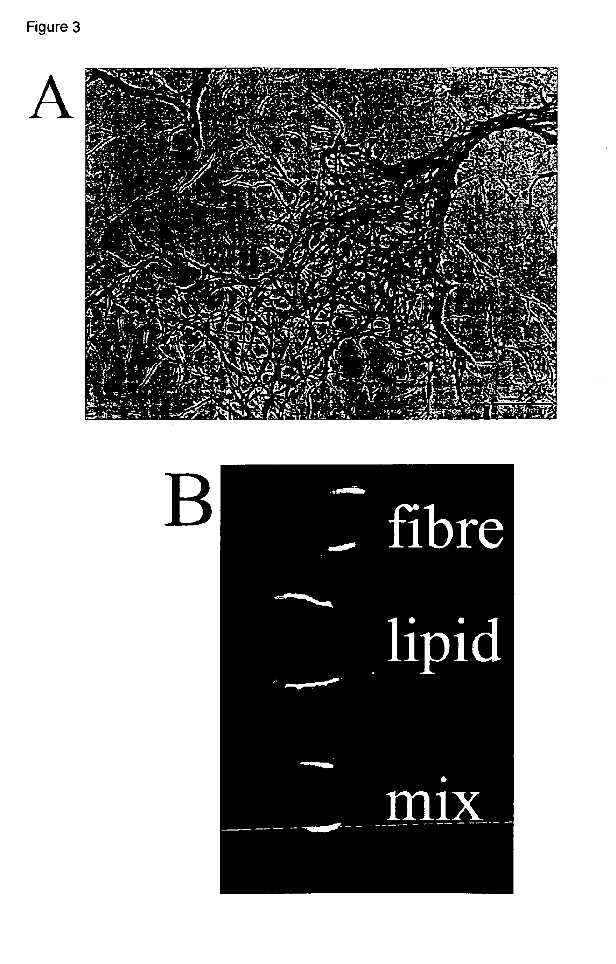

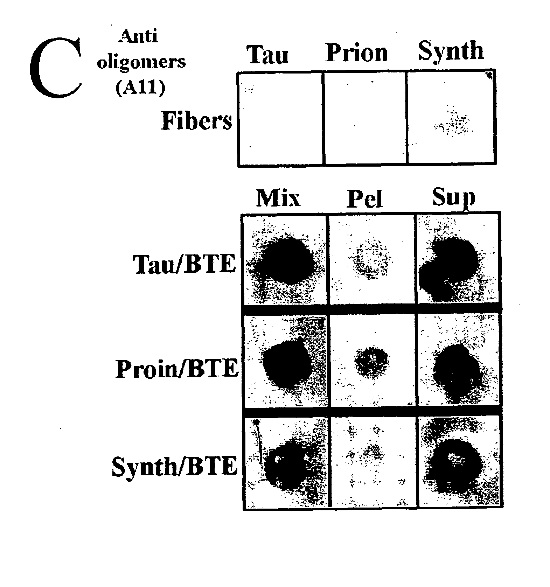

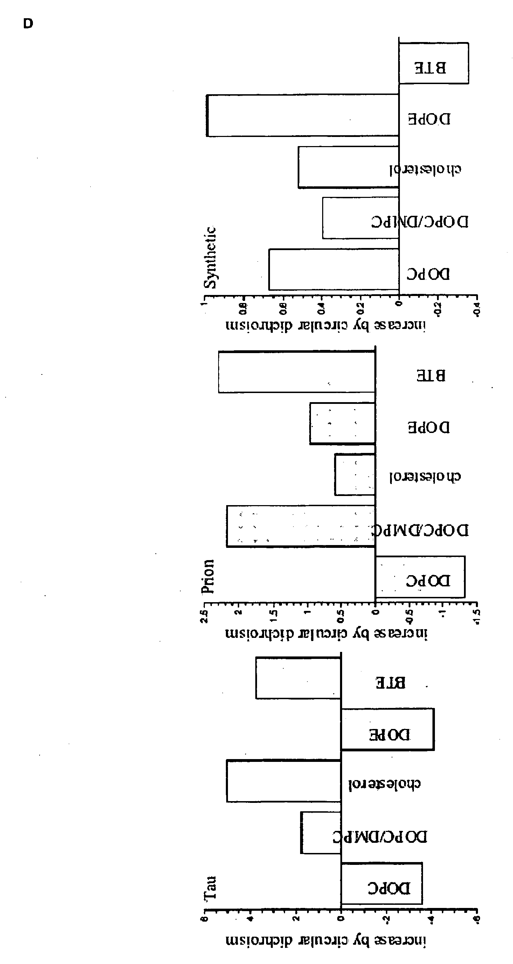

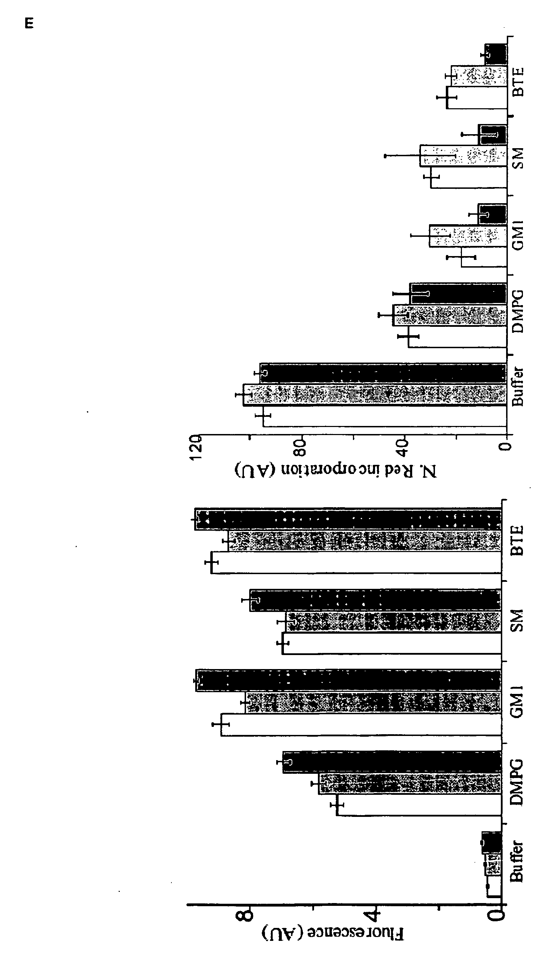

Figure 3: Lipids affect a wide range of amyloid fibers. (A) Electron microscopy images of mature amyloid fibers consisting of a hexapeptide sequence derived from tau (NH₂-KVQIIN-COOH), mixed with liposomes consisting of DOPC. (B) Photograph of a 50 µL droplet of the stock preparations of amyloid fiber and liposomes as well as of the corresponding mixture of amyloid and lipid. The separation into a soluble and insoluble phase is readily apparent. (C) Dot blot of hexapeptide fibrils/BTE mixtures and the soluble and pellet fractions thereof, probed with the oligomer specific antibody A11 (D) Circular dichroism spectra for indicated lipids and hexapeptide sequences demonstrating lipid-peptide interaction. (E) Quantification of cytotoxicity. Neutral red incorporation by neurons treated with the soluble fraction of the mixture between hexapeptide amyloid fibers and liposomes containing the indicated lipids. Data for the tau hexapeptide NH₂-KVQIIN-COOH (white), prion hexapeptide NH₂-ISFLIF-COOH (light gray) and the synthetic peptide NH₂-STVIIE-COOH (dark grey) are shown (left). On the right, the fluorescence intensity of annexin V staining is shown for the same samples.

**Table 1: Comparison of the toxicity of SEC fractions of Aβ42 mixed with various lipids. The SEC fractionation of the soluble part of Aβ42 fiber/lipid mixtures typically shows two peaks with a significant amount of Aβ42 oligomers, eluting at 16.7 and 19.3 mL. Toxicity to neuronal cells of the various lipid emulsions is quantified for each peak by neutral red incorporation. A qualitative indication of binding to the oligomer specific A11 antibody is also given.**

| ***Lipid*** | ***Fraction*** | ***N.Red*** | ***A11 binding*** |
|---|---|---|---|
| Pi | 16.7 | 89.5 ± 2.5 | + |
| | 19.3 | 90.9 ± 1.2 | + |
| DMPG | 16.7 | 53.0 ± 6.2 | ++ |
| | 19.3 | 67.9 ± 3.7 | ++ |
| Chol | 16.7 | 50.5 ± 3.5 | +++ |
| | 19.3 | 64.7 ± 3.8 | ++ |
| SM | 16.7 | 43.1 ± 2.4 | +++ |
| | 19.3 | 63.9 ± 6.6 | +++ |
| GM1 | 16.7 | 36.1 ± 2.9 | +++ |
| | 19.3 | ND | ++ |
| BTE | 16.7 | 25.2 ± 4.3 | +++ |
| | 19.3 | 49.4 ± 3.4 | +++ |

### Aims and detailed description of the invention

A variety of diseases result because of misfolded protein that deposits in extracellular space in the body. These deposits can be amorphous (disordered) or fibrillar (ordered). Inclusion bodies are an example of amorphous aggregates, and amyloid fibril is an example of fibrillar or ordered aggregates. Diseases caused by fibrillar aggregate deposits or amyloid fibrils are called amyloidosis or amyloidogenic diseases. Amyloid deposits can be formed extracellularly or intracellularly. The following is a non-limiting list of proteins followed parenthetically by associated diseases of which proteins can assemble into an amyloid fibril confirmation: a mixture of amyloid-beta-40 and amyloid beta-42 peptide (amyloid plaques in Alzheimer's Disease and cerebral amyloid angiopathy), tau (neurofibrillary tangles in Alzheimer's disease, frontotemporal dementia and Pick's disease), prion protein, PrP (spongiform encephalopathies such as Creutzfeld-Jacob disease, bovine spongiform encephalopathy, fatal familial insomnia, Gerstmann-Straussler disease, Huntington disease like-1 and kuru), superoxide dismutase (amyotrophic lateral sclerosis), alpha-synuclein (Lewy bodies in Parkinson's disease), islet amyloid polypeptide (Diabetes Type II), IgG light chain (multiple myeloma plasma cell dyscrasias and primary systemic amyloidosis), transthyretin (familial amyloidotic polyneuropathy and senile systemic amyloidosis), procalcitonin (medullary carcinoma of thyroid, beta₂-microglobulin (chronic renal failure), atrial natriuretic factor (congestive heart failure), serum amyloid A (chronic inflammation), Apolipoprotein A1 and A2 (hereditary systemic amyloidosis and atherosclerosis), gelsolin (familial amyloidosis), huntingtin (Huntington's disease), lysozyme (autosomal dominant hereditary amyloidosis), medin or lactadherin (aortic medial amyloidosis), insulin (injection localized amyloidosis), amyloid Adan/ABri peptide (familial British and Danish dementia), fibrinogen alpha-A (hereditary renal amyloidosis), ataxin-3 (Machado-Joseph disease or spinocerebellar ataxia-3), TATA boxbinding protein (spinocerebellar ataxia type 17) and cystatin C (hereditary cerebral hemorrhage with amyloidosis and hereditary renal amyloidosis). Each amyloid fibril (or fiber) deposit formed from a different protein causes a different disease by affecting a different organ or tissue in the body. However, the characteristics of different amyloid fibrils, namely structure and morphology, observed by electron microscopy and X-ray fiber diffraction appear to be quite similar in nature. In the present invention we have developed a process to produce soluble amyloid oligomers derived from insoluble, inert amyloid fibers.

Accordingly the invention provides a process for the production of amyloid oligomers from amyloid fibers comprising contacting said amyloid fibers with at least one lipid. Amyloid fibers consist of proteins mentioned herein before such as amyloid beta, tau, superoxide dismutase, huntingtin, prion protein, alpha-synuclein. Alternatively amyloid fibers consist of fragments of said proteins (e.g. peptides). Amyloid fibers can also be derived from allelic variants or mutants of said proteins. Fragments can be made recombinantly or fragments can be synthetic peptides. Amyloid fiber formation is induced by dissolving such peptides in aqueous buffers of a suitable pH (depending on the charged residues: low pH is sometimes required to neutralise glutamate and aspartate) at elevated concentrations (typically 0.2 and 2 mM, but this is not limiting). The at least one lipid can be directly mixed with amyloid fibers. In a preferred embodiment at least one lipid is administered to amyloid fibers when incorporated into a vesicle. In another preferred embodiment at least one lipid is administered to amyloid fibers when incorporated into a liposome. In order to produce liposomes of any kind, lipids need to be introduced into an aqueous environment. When dry lipid films are exposed to mechanical agitation in such an aqueous environment, large multilamellar vesicles are spontaneously formed. In order to produce smaller, uniformly sized and unilamellar vesicles (herein called liposomes in the examples), additional energy has to be dissipated into the system. The latter is often achieved by mechanical extrusion or by sonication. A general overview to manufacture liposomes is incorporated here by reference (Mozafari Reza M (2005) Cellular & Molecular Biology Letters 10, 711-719). A lipid can be a biological lipid or a synthetic lipid. Non-limiting examples of lipids which can be used are gangliosides, sphingomyelins, cholesterol, dioleoylphosphatidylcholine (DOPC), dioleoyl-phosphatidyiserine (DOPS), dimyristoylphosphatidylcholine (DMPC), dimyristoylphosphatidylglycerol (DMPG), phosphatidylethanolamine (DSPE) and dioleoylphosphatidylethanolamine (DOPE). In another embodiment the lipid is a membrane extract of biological cells (e.g. brain extract). Amyloid oligomers are soluble, detergent-stable configurations of more than one amyloid protein which are not amyloid in nature.

In a particular embodiment amyloid oligomers prepared from amyloid fibers which consist of tau or amyloid beta or the prion protein are toxic for cells, in particular neuronal cells. Neuronal cells comprise sensory neurons, motor neurons and hippocampal neurons.

In yet another particular embodiment the invention envisages the use of the amyloid oligomers, in particular the amyloid beta oligomers, for the generation of a non-human Alzheimer's disease model. In yet another particular embodiment said non-human Alzheimer's disease model is generated by intracerebroventricular injection of a non-human animal. Suitable animals comprise rabbits, mice and rats.

In yet another embodiment the invention provides an *in vivo* screening method to identify compounds that interfere with the toxicity of amyloid oligomers comprising a) contacting amyloid oligomers produced according to the present invention with at least one compound, b) determining the toxicity of the complex formed in step a) on cells and c) identifying at least one compound that interferes with the toxicity of said amyloid oligomers on the cells. A cell can be any biological cell. Cells are preferentially neuronal cells.

Compounds comprise peptides, tetrameric peptides, proteins and small molecules. Small molecules, e.g. small organic molecules, can be obtained, for example, from combinatorial and natural product libraries. The determination of the toxicity of the amyloid oligomers, e.g. the cytotoxicity, can for example be determined by measuring the inhibition of cell growth, by measuring the cellular necrosis, by measuring the cellular apoptosis. Several viability assays known in the art can be used such as the neutral red incorporation assay, annexin V staining, propidium iodide staining and caspase-3 staining.

In yet another embodiment the invention provides an *in vitro* screening method to identify compounds that interfere with the formation of amyloid oligomers comprising a) forming amyloid oligomers according to the method of the present invention in the presence of at least one compound, b) detecting the inhibition of the formation of amyloid oligomers and c) identifying at least one compound that interferes with the formation of amyloid oligomers. The formation of amyloid oligomers can be measured in vitro by several methods such as electron microscopy, in immunoblots by using an oligomer-specific antibody, by size exclusion chromatography, by circular dichroism spectroscopy, by Fourier Transform Infrared spectroscopy, by ultracentrifugation and by dynamic light scattering experiments.

### Examples

### 1. The formation of soluble amyloid oligomers from insoluble amyloid fibers

Amyloid-beta-42 was incubated for one week at 1 mg/mL in 50mM Tris-HCl pH 7.4 and controlled the presence of amyloid-beta-42 fibers by electron microscopy. These mature amyloid-beta-42 (Aβ42) fibers were subsequently harvested by centrifugation and added to primary hippocampal mouse neurons and differentiated N2A cells at a final concentration of 5µM. As expected, these mature fibers were largely inert, displaying only modest neurotoxicity. Upon addition of 250 µg/mL liposomes composed of mixtures of various membrane lipids (including gangliosides, sphingomyelins and cholesterol) to 1 mg/mL mature Aβ42 fibers an extremely toxic emulsion was generated as measured by different viability assays, including the morphological shape change of the cells, neutral red incorporation assay, annexin V staining, propidium iodide staining and caspase-3 staining. Importantly, the lipid preparations (and the inert amyloid fibrils) alone were not toxic. The toxic Aβ42 fiber/lipid emulsion partitioned into two phases which were separated by centrifugation for 20 min at 14000g. The supernatant fraction was for all lipids tested significantly more toxic to neuronal cells than the total Aβ42 /lipid mixtures, whereas the pellet was largely inert, showing lipid-induced formation of soluble toxic species from mature Aβ42 fibers. This was confirmed by electron microscopy (Figure 2A), by immunoblots demonstrating reactivity with the oligomer-specific A11 antibody, by size exclusion chromatography, by circular dichroism spectroscopy, by Fourier Transform Infrared spectroscopy, by ultracentrifugation and by dynamic light scattering experiments. All assays confirmed the generation of Abeta oligomers in the supernatant of lipid/fiber emulsions (Figure 2).

### 2. Characterisation of amyloid beta oligomers

In a next step we proceeded to the characterisation of these fibril-lipid mixtures. Transmission electron microscopy revealed that amyloid fibrils were converted by lipids to an insoluble fraction containing fractured and highly intertwined amyloid material surrounded by short amyloid fragments, whereas the supernatant contained protofibrillar structures, confirming fibril destabilisation and resolubilisation in the presence of lipids. Confocal microscopy using immunostaining with the antibody A11 that is specific for 'soluble prefibrillar oligomers', shows a granular decoration of material on the plasma membrane of primary neurons but also significant internalization matching the behavior of prefibrillar toxic material extracted from AD brains. Amyloid-lipid emulsions were further deposited under a sucrose gradient and centrifuged at 100.000g for 1 hour. We used the Abeta specific mAb 6E10 and the oligomer-specific pAB A11 to detect Abeta species. Whereas both the top of the gradient and the pellet reacted with 6E10, only the top of the gradient reacted with A11, demonstrating that fibrils are indeed resolubilised, and that the soluble fraction migrates in the same fraction as the liposomes, whereas insoluble amyloid material was pelleted. Dynamic light scattering (DLS) at a detection angle of 90° relative to the incident beam, detected hydrodynamic radii between 10 and 100 µm in samples of mature fibrils, fitting to a spherical model). When lipids were added to the sample the hydrodynamic radius dropped to a range between 100 nm and 1 µm, indicating significant heterogeneity. A similar size distribution is observed from light scattering measured at 173° (back scattering), excluding misinterpretations due to the angular dependence of light scattering. Both the size distribution and heterogeneity observed by light scattering are in excellent agreement with sizes observed by electron microscopy, where flexible protofibrils are observed to curl into spheroid shapes with dimensions between 100-300 nm. Further confirmation that the amyloid fibrils revert to a protofibrillar state, comes from spectroscopic analysis, which showed intermolecular beta or cross-beta structure similar to that of mature amyloid fibrils. Circular dichroism (CD) revealed an increase in the amplitude around 220 nm, but no significant shape change compared to the amyloid far UV spectrum, indicating an increase in soluble material in the amyloid-lipid mixtures with a similar beta-sheet content as the amyloid fibrils. Fourier-transform infrared (FTIR) spectra indicated that lipid-induced protofibrils possess a similar intermolecular beta-extended structure as mature fibrils (corresponding to the spectral band at 1623 cm⁻¹), but the difference FTIR spectrum revealed some degree of unfolding in the protofibrils as compared to the mature amyloid fibrils, as was apparent from the 1647 cm⁻¹ band. We proceeded to analyse our lipid-induced protofibrils by Size Exclusion Chromatography (SEC). When the supernatant of a lipid/fibril mixture was injected on a S75/HR10 column, a single peak at 15.8 mL was eluted which immunostained with both the 6E10 and A11 antibodies. Size determination from the elution volume yields an apparent molecular weight of approximately 9kD (dimeric Abeta). This estimation, however, is only valid for globular proteins that do not interact with the column matrix. These requirements are certainly not met here as the analysis of the elution peak by TEM again clearly shows a heterogenous mixture of protofibrillar oligomers with a size of 100-200 nm. To characterize the size distribution of the lipid/fibril mixture we instead utilised an 18-angles static light scattering (SLS) detector inline with the SEC column, which allows to infer size information directly from the angular dependence of the scattered light intensity in an absolute manner that is independent from shape or gel matrix interactions. SLS indicates a strong non-linear angle dependence in the light scattering intensity, consistent with objects larger than 100 nm, and a calculated molecular weight of 80-500 kDa (between 20 and 90 monomeric units). The fact that a heterogeneous sample elutes as a focused peak is consistent with strong interactions with the gel matrix, since under these conditions the elution profile is no longer determined by the size but by the strength of the column interactions and no size separation is achieved. In all, the methods used here are in agreement with earlier analysis of the structure and toxicity of protofibrils, and show that the product cannot be defined by a single molecular mass.

### 3. Lipid specificity of the process to generate amyloid oligomers

We further analyzed the lipid specificity of Aβ42 fiber disassembly and neurotoxicity by mixing the Aβ42 fibers with liposomes of several compositions (Figure 1). Interestingly the strongest neurotoxicity was triggered when Aβ42 amyloid fibrils were subjected to liposomes enriched in ganglioside GM1 or sphingomyelin. For all lipids, the neurotoxic material eluted in the same SEC fractions at 16.3 and 19.6 mL (Table 1). Interestingly, a similar degree of neurotoxicity was also obtained with Aβ42 amyloid fibers exposed to total membrane extracts from brain. Finally we found that neurotoxicity was also induced by other phospholipids including DOPC, DMPG and DOPE. We conclude that Aβ fibers are efficiently disassembled by different lipids into toxic oligomers.

### 4. Formation of amyloid oligomers derived from tau, human prion and synthetic amyloid fibers

Finally, we extended our observations to other amyloid fibers, using previously characterized amylogenic hexapeptides derived from Tau (NH₂-KVQIIN-COOH) and the human prion protein (NH₂-ISFLIF-COOH). We also used an artificial amylogenic sequence (NH₂-STVIIE-COOH) that was designed *in silico* and that has no role in disease. Again, addition of phospholipids to the biologically inert, mature amyloids generated from these hexapeptides induced dramatic cytotoxicity in primary neurons (Figure 3). Fiber disassembly was observed by electron microscopy, circular dichroism and dynamic light scattering (Figure 3). The lipid specificity displayed by amyloid fibers from different peptides varied slightly (Figure 3).

### 5. In vitro assay to screen for molecules able to interfere with the release of toxic oligomers from amyloid fibers

One specific detection technique for toxic oligomers of amyloid-beta is by use of the A11 antibody (Kayed R et al (2003) Science 300, 486-9). Alternatively a colorimetric prescreening can be performed which detects soluble peptides released from amyloid-beta fibers followed by detection with the A11 antibody. Several mixtures of amyloid-beta are currently evaluated (10/1 and 7/3 mixtures of amyloid-beta40/ amyloid-beta42 are physiologically most relevant)

### Typical A11 assay:

### Stock solutions:

Basic buffer: 50 mM Tris-HCl pH 7.5, 100 mM NaCl; Extraction buffer: 50 mM Tris-HCl pH 7.5, 100 mM NaCl, 2 mM EDTA, 0.1 % SDS; Abeta fibers: 200 µM amyloid-beta, incubated for 10 days at room temperature in basic buffer; Liposomes: 2 mg/mL total lipid concentration, (100 DOPC, 100 DMPC, 100 Chol, 50 BTE).

### Protocol:

Mix 9 µL basic buffer + 5 µL Amyloid beta fibers + 5 µL liposomes + 1 µL of a compound solution; Incubate shaking overnight at RT (600 rpm, at least); Add 5x extraction buffer, incubate shaking 30 minutes; Centrifuge 13600 rpm in benchtop (eppendorf) centrifuge for 15 minutes; Dotblot supernatant (using vacuum dotblotting apparatus); Wash + Block with milk; Incubate with 1:2000 A11 antibody overnight at 4 °C; Wash 2x; Incubate with secondary antibody 30 min at room temperature; Develop and detect the presence of amyloid-beta oligomers. Compounds that interfere with amyloid-beta oligomer formation give a reduced signal.

### Prescreening colorimetric assay:

Stock solutions: Basic buffer: 50 mM Tris-HCl pH 7.5, 100 mM NaCl; Extraction buffer: 50 mM Tris-HCl pH 7.5, 100 mM NaCl, 2 mM EDTA, 0.1 % SDS; Fluorescent Abeta fibers: 200 µM N-terminally labelled Ab, incubated for 10 days at room temperature (RT) in basic buffer. The fluorescent labelling kit of Alexa dyes from invitrogen is used since labelling does not interfere with the fiber formation (fibrillisation); Liposomes: 2 mg/mL total lipid concentration, (100 DOPC, 100 DMPC, 100 Chol, 50 BTE) Protocol: Mix 9 µL basic buffer + 5 µL Ab fibers + 5 µL liposomes + 1 µL of a compound solution; Incubate shaking overnight at RT (600 rpm, at least); Add 5x extraction buffer, incubate shaking 30 minutes; Centrifuge 13600 rpm in benchtop (eppendorf) centrifuge for 15 minutes; Transfer supernatant into 80 µL basic buffer, mix well; Measure Alexa dye fluorescence using plate reader. Compounds that interfere with amyloid-beta oligomer formation give a reduced signal.

### 6. In vivo assay to screen for molecules able to interfere with the cytotoxicity of amyloid fibers

Non-fluorescent fibers are prepared as in example 5. Cells can be for example HELA cells or hippocampal neurons. Cells are pretreated with compounds before toxic amyloid-beta oligomers are added. The inhibition of toxicity is monitored by use of for example MTT staining, neutral red staining or annexin staining.

### 7. Toxicity of amyloid oligomers

We incubated Abeta-42 for one week at 1 mg/mL in 50mM Tris-HCl pH 7.4 and controlled the presence of Abeta-42 fibers by electron microscopy. These mature Abeta-42 fibers were subsequently harvested by centrifugation and added to primary hippocampal mouse neurons and differentiated N2A cells at a final concentration of 5µM. As expected, these mature fibers were largely inert, displaying only modest neurotoxicity. Upon addition of 250 µg/mL liposomes composed of mixtures of various membrane lipids (including gangliosides, sphingomyelins and cholesterol) to 1 mg/mL mature Abeta-42 fibers an extremely toxic emulsion was generated as measured by different viability assays, including the morphological shape change of the cells, neutral red incorporation assay, annexin V staining, propidium iodide staining and caspase-3 staining. Importantly, the lipid preparations (and the inert amyloid fibrils) alone were not toxic. The toxic Abeta-42 fiber/lipid emulsion partitioned into two phases which could be separated by centrifugation for 20 min at 14000g. The supernatant fraction was for all lipids tested significantly more toxic to neuronal cells than the total Abeta-42 /lipid mixtures, whereas the pellet was largely inert, suggesting lipid-induced formation of soluble toxic species from mature Abeta-42 fibers. To further characterize the physiological relevance of lipid-induced oligomers, we proceeded to single intraventricular injection (2.5 µl) of supernatants from amyloid-lipid mixtures in the brain of adult mice. Immunostaining of brain samples with the 6E10 antibody demonstrated the effective delivery of Abeta to the brain and subsequent distribution away from the injection spot to cortex and hippocampus within 90 min after injection. We evaluated the biological effects in various exploratory and memory/learning tests, all within 40-90 min after the injection. In open field tests the injected animals appeared extremely hyperactive and hypermobile. This was substantiated by measuring the total length of path and number of crosses of the center that were both significantly higher than in the animals injected with lipid samples or pure mature amyloid fibrils alone. Fear conditioning using light-dark passive avoidance tests in combination with electrical shock was possible, but animals injected with oligomers did not succeed to form memory at all as measured 24 hours later by delay before entering the dark room. Also contextual fear conditioning and auditory-cue fear conditioning as measured by typical freezing behavior 24 hours after conditioning was severely disturbed in mice exposed to oligomers, in contrast to the control mice exposed to lipid or mature amyloid alone. Of interest after 1 week the mice injected with oligomers recovered completely and were not different in behaviour from the control treated or untreated animals. Also preliminary analysis of brains injected with oligomers revealed little staining for apoptotic markers. Thus, a single injection of oligomers causes only transient and most likely functional effects but no significant irreversible neurotoxicity. This agrees with other studies showing that forward oligomers have immediate but transient effects on synaptic function.

### Materials and methods

### Chemicals

Alzheimer beta peptides 1-40 and 1-42 were purchased from Sigma-Aldrich. All purified and synthetic lipids were obtained from Avanti Lipids (USA). Model hexapeptides were obtained from Jerini Peptide Technologies (Germany). Uranyl acetate was obtained from BDH.

### Preparation of lipid vesicles and liposomes

All lipids were obtained from Avanti Polar Lipids (USA) except the ganglioside GM1 which was obtained from Larodan Chemicals (Sweden). The stock concentration was 20 mg/mL in chloroform. The various lipid mixtures discussed in the paper were prepared in Corex roundbottom glass tubes, dried under a gentle N2 stream and resuspended in 400 µL diethylether for 10 min at room temperature after which they were quickly dried in a water bath at 50 °C. The resulting film was placed under vacuum for 1 h to remove trace solvent and rehydrated in 800 µL of 50 mM Tris pH 7.5, 1 mM EDTA, 0.1 mM NaCl. The resulting vesicles suspension was allowed to stabilize for 1 h at room temperature, sonicated for 20 seconds (Branson sonifier) and extruded 15 times with an Avanti mini-extruder (Avanti Polar Lipids, USA). This suspension was purified on an S75 gel filtration column using an Akta system from GEHealthcare (UK). The approximate lipid concentration in the stock preparation was 10 mg/mL.

### Preparation of amyloid fibers and amyloid/lipid mixtures

Amyloid fibers of the Alzheimer beta-peptide 1-40 and 1-42 were obtained by incubation of 200 mM peptide solution in 50 mM Tris pH 7.5 for 1 week at room temperature. Amyloid fibrils of the hexapeptides were obtained by incubation at 1 mM peptide in 20 mM Tris-glycine pH 2.6 during a minimum of 1 week at room temperature. Amyloid fiber/lipid mixtures were prepared by mixing fibril and liposome stock solutions 1:1 and incubating for 1-12 hours at room temperature, shaking at 700 rpm.

### Immunodetection of Aβ and oligomers

Fractions of volume 20-30uL were spotted onto nitrocellulose membrane in 5uL overlays with drying in between applications. Membranes were blocked for one hour in blocking buffer (PBS, 0.1% Tween-20 (PBST) and 5% fat free milk). Membranes were incubated overnight at 4°C with rabbit anti-oligomer (A11) antibody (Invitrogen) diluted 1:1000 in blocking buffer. Following 3 x 10 mins washes in PBST, membranes were incubated for 30mins in anti-rabbit HRP (Promega) antibody diluted 1:5000 in PBST. Membranes were washed, incubated briefly in the chemiluminescence substrate WestDura (Pierce) and visualised via CCD camera using a BioRad ChemiDoc XRS (20 second exposure). Membranes were then stripped by 3 x 10min washes with stripping buffer (50mM Glycine, 500mM NaCl, 0.1 % NP40, pH 2.4) and reincubated in WestDura to ensure no signal could be detected. Membranes were then reblocked and probed as described above with mouse anti-beta amyloid (6E10) antibody (Abcam) diluted 1:2000 and anti-mouse HRP (Promega) antibody diluted 1:5000.

### Size Exclusion Chromatography (SEC)

SEC was performed using a Superdex75 column from GEHealthcare (UK) on a AKTA purifier 10 system using a flowrate of 0.4 mL/min in the following running buffer: 50 mM Tris pH 7.5, 0.1 % SDS, 150 mM NaCl, 1 mM EDTA. Fractions of 0.5 mL were automatically collected using by the AKTA system. Synthetic plaques were mixed 1:5 with 5 times concentrated buffer for 30 minutes prior to injection and the samples were filtered using 0.22 µm spin-X centrifuge tube filters (Corning). Samples of 200 µL were injected per run and the total monomeric peptide concentration was 50 µM.

### Electron Microscopy (EM)

Aliquots (5 µL) of the aggregate preparation were adsorbed to carbon-coated FormVar film on 400-mesh copper grids (Piano GmbH, Germany) for 1 min. The grids were blotted, washed twice in droplets of Milli-Q water, and stained with 1% (wt/vol) uranyl acetate.

After drying in vacuum O/N, samples were studied with a FEI Morgagni™ 268(D) microscope at 120 kV and a JEOL JEM-2100 microscope at 200 kV.

### Spectroscopic analysis and ultracentrifugation

### CD measurements were recorded on a Jasco Spectropolarimeter J715 using quartz cuvettes (Hellma) with path lengths ranging from 0.2 to 0.5mm. A scan rate of 1 nm/s was used and 15 spectra were averaged for each measurement. Samples were thermostatted at 25 °C using a waterbath. Dynamic Light Scattering (DLS) was recorded on a Spectroscatter 201 apparatus (RiNA GmbH, Germany), using the Photomeasure software package (v3.01 p17). Static Light Scattering and refractive index data were collected continuously during SEC fractionation, using a Dawn Heleos and Optilab rEX from Wyatt (USA) that were connected inline to the AKTA system. Weight-averaged molecular, z-average radius of gyration and z-average hydrodynamic radius values were calculated using the ASTRA software package. Fourier Transform Infrared Spectroscopy was performed on a Bruker Tensor 37 FT-IR spectrometer equipped with an AquaSpec flowcell. The floating assay was performed by layering a 60% sucrose gradient on top of a fiber/lipid mixture followed by centrifugation at 150.000g for 1 h, during which liposomes travel to the top of the gradient. Three samples were taken: from the top (liposomes), the bottom (pelleted material) and the middle (gradient).

### Cell culture

Primary hippocampal neurons were generated and processed for immunohistochemistry as previously documented³⁶.

### References

1. Kang, J. et al. The precursor of Alzheimer's disease amyloid A4 protein resembles a cellsurface receptor. Nature 325, 733-6 (1987).
2. Ropper, A. H. & Williams, R. S. Relationship between plaques, tangles, and dementia in Down syndrome. Neurology 30, 639-44 (1980).
3. Glenner, G. G. & Wong, C. W. Alzheimer's disease and Down's syndrome: sharing of a unique cerebrovascular amyloid fibril protein. Biochem Biophys Res Commun 122, 1131-5 (1984).
4. Van Broeckhoven, C. et al. Amyloid beta protein precursor gene and hereditary cerebral hemorrhage with amyloidosis (Dutch). Science 248, 1120-2 (1990).
5. Levy, E. et al. Mutation of the Alzheimer's disease amyloid gene in hereditary cerebral hemorrhage, Dutch type. Science 248, 1124-6 (1990).
6. Chartier-Harlin, M. C. et al. Early-onset Alzheimer's disease caused by mutations at codon 717 of the beta-amyloid precursor protein gene. Nature 353, 844-6 (1991).
7. Goate, A. et al. Segregation of a missense mutation in the amyloid precursor protein gene with familial Alzheimer's disease. Nature 349, 704-6 (1991).
8. Murrell, J., Farlow, M., Ghetti, B. & Benson, M. D. A mutation in the amyloid precursor protein associated with hereditary Alzheimer's disease. Science 254, 979 (1991).
9. Naruse, S. et al. Mis-sense mutation Val-Ile in exon 17 of amyloid precursor protein gene in Japanese familial Alzheimer's disease. Lancet 337, 978-9 (1991).
10. Scheuner, D. et al. Secreted amyloid beta-protein similar to that in the senile plaques of Alzheimer's disease is increased in vivo by the presenilin 1 and 2 and APP mutations linked to familial Alzheimer's disease. Nat Med 2, 864-70 (1996).
11. Hardy, J. A. & Higgins, G. A. Alzheimer's disease: the amyloid cascade hypothesis. Science 256, 184-5 (1992).
12. Hardy, J. & Selkoe, D. J. The amyloid hypothesis of Alzheimer's disease: progress and problems on the road to therapeutics. Science 297, 353-6 (2002).
13. Rovelet-Lecrux, A. et al. APP locus duplication causes autosomal dominant early-onset Alzheimer disease with cerebral amyloid angiopathy. Nat Genet 38, 24-6 (2006).
14. Theuns, J. et al. Promoter mutations that increase amyloid precursor-protein expression are associated with Alzheimer disease. Am J Hum Genet 78, 936-46 (2006).
15. Lue, L. F. et al. Soluble amyloid beta peptide concentration as a predictor of synaptic change in Alzheimer's disease. Am J Pathol 155, 853-62 (1999).
16. McLean, C. A. et al. Soluble pool of Abeta amyloid as a determinant of severity of neurodegeneration in Alzheimer's disease. Ann Neurol 46, 860-6 (1999).
17. Wang, J., Dickson, D. W., Trojanowski, J. Q. & Lee, V. M. The levels of soluble versus insoluble brain Abeta distinguish Alzheimer's disease from normal and pathologic aging. Exp Neurol 158, 328-37 (1999).
18. Naslund, J. et al. Correlation between elevated levels of amyloid beta-peptide in the brain and cognitive decline. Jama 283, 1571-7 (2000).
19. Citron, M. et al. Mutant presenilins of Alzheimer's disease increase production of 42residue amyloid beta-protein in both transfected cells and transgenic mice. Nat Med 3, 67-72 (1997).
20. Crook, R. et al. A variant of Alzheimer's disease with spastic paraparesis and unusual plaques due to deletion of exon 9 of presenilin 1. Nat Med 4, 452-5 (1998).
21. Houlden, H. et al. Variant Alzheimer's disease with spastic paraparesis and cotton wool plaques is caused by PS-1 mutations that lead to exceptionally high amyloid-beta concentrations. Ann Neurol 48, 806-8 (2000).
22. Cleary, J. P. et al. Natural oligomers of the amyloid-beta protein specifically disrupt cognitive function. Nat Neurosci 8, 79-84 (2005).
23. Walsh, D. M. et al. The role of cell-derived oligomers of Abeta in Alzheimer's disease and avenues for therapeutic intervention. Biochem Soc Trans 33, 1087-90 (2005).
24. Klyubin, I. et al. Amyloid beta protein immunotherapy neutralizes Abeta oligomers that disrupt synaptic plasticity in vivo. Nat Med 11, 556-61 (2005).
25. Lambert, M. P. et al. Diffusible, nonfibrillar ligands derived from Abeta1-42 are potent central nervous system neurotoxins. Proc Natl Acad Sci U S A 95, 6448-53 (1998).
26. Lesne, S. et al. A specific amyloid-beta protein assembly in the brain impairs memory. Nature 440, 352-7 (2006).
27. Carrell, R. W. Cell toxicity and conformational disease. Trends Cell Biol 15, 57480 (2005).
28. Aggeli, A. et al. Hierarchical self-assembly of chiral rod-like molecules as a model for peptide beta -sheet tapes, ribbons, fibrils, and fibers. Proc Natl Acad Sci U S A 98, 11857-62 (2001).
29. Makin, O. S., Atkins, E., Sikorski, P., Johansson, J. & Serpell, L. C. Molecular basis for amyloid fibril formation and stability. Proc Natl Acad Sci U S A 102, 315-20 (2005).
30. Cannon, M. J., Williams, A. D., Wetzel, R. & Myszka, D. G. Kinetic analysis of beta-amyloid fibril elongation. Anal Biochem 328, 67-75 (2004).
31. Kayed, R. et al. Common structure of soluble amyloid oligomers implies common mechanism of pathogenesis. Science 300, 486-9 (2003).
32. Lacor, P. N. et al. Synaptic targeting by Alzheimer's-related amyloid beta oligomers. J Neurosci 24, 10191-200 (2004).
33. Kim, S. I., Yi, J. S. & Ko, Y. G. Amyloid beta oligomerization is induced by brain lipid rafts. J Cell Biochem (2006).
34. Kakio, A., Nishimoto, S., Yanagisawa, K., Kozutsumi, Y. & Matsuzaki, K. Interactions of amyloid beta-protein with various gangliosides in raft-like membranes: importance of GM1 ganglioside-bound form as an endogenous seed for Alzheimer amyloid. Biochemistry 41, 7385-90 (2002).
35. Hayashi, H. et al. A seed for Alzheimer amyloid in the brain. J Neurosci 24, 4894902 (2004).
36. Cupers, P. et al. The discrepancy between presenilin subcellular localization and gamma-secretase processing of amyloid precursor protein. J Cell Biol 154, 731-40 (2001).

## Claims

1. An in vitro process for producing amyloid oligomers from amyloid fibers comprising the formation of a lipid-amyloid fiber emulsion by contacting said amyloid fibers with at least one lipid.

2. The process according to claim 1 wherein said at least one lipid is a biological lipid such as a ganglioside, sphingomyelin or cholesterol.

3. The process according to claims 1 or 2 further comprising the centrifugation of the said emulsion and retaining the supernatant fraction.

4. The process according to any one of claims 1 to 3 wherein said amyloid fibers consist of amyloid beta, tau, prion protein or a fragment thereof.

5. The process according to claim 4 wherein the resulting amyloid oligomers are toxic for neuronal cells.

6. An *in vivo* screening method in a non-human animal to identify compounds that interfere with the toxicity of amyloid oligomers comprising a) contacting the amyloid oligomers obtainable by a process according to any one of claims 1 to 5 with at least one compound, b) determining the toxicity of the complex formed in step a) on cells and c) identifying at least one compound that interferes with the toxicity of said amyloid oligomers.

7. The in vivo screening method according to claim 6 wherein said cells are neuronal cells.

8. An *in vitro* screening method to identify compounds that interfere with the formation of amyloid oligomers comprising a) forming amyloid oligomers as in claims 1, 2, 3 or 4 in the presence of at least one compound, b) detecting the inhibition of the formation of amyloid oligomers and c) identifying at least one compound that interferes with the formation of amyloid oligomers.

9. The method according to claim 8 wherein said detection step b) is spectrophotometric.

## Patentansprüche

1. In-vitro-Verfahren zur Herstellung von Amyloid-Oligomeren aus Amyloidfasern, wobei das Verfahren das Bilden einer Lipid-Amyloidfaser-Emulsion durch Inkontaktbringen der Amyloidfasern mit mindestens einem Lipid umfasst.

2. Verfahren nach Anspruch 1, wobei das mindestens eine Lipid ein biologisches Lipid ist, wie Gangliosid, Sphingomyelin oder Cholesterin.

3. Verfahren nach Anspruch 1 oder 2, das weiterhin das Zentrifugieren der Emulsion und das Aufbewahren der Überstandsfraktion umfasst.

4. Verfahren nach einem der Ansprüche 1 bis 3, wobei die Amyloidfasern aus Beta-Amyloid, Tau, Prionprotein oder einem Fragment davon bestehen.

5. Verfahren nach Anspruch 4, wobei die resultierenden Amyloid-Oligomere für neuronale Zellen toxisch sind.

6. In-vivo-Screening-Verfahren in einem nichtmenschlichen Tier zur Identifizierung von Verbindungen, die die Toxizität von Amyloid-Oligomeren stören, wobei das Verfahren a) das Inkontaktbringen der Amyloid-Oligomere, die durch ein Verfahren nach einem der Ansprüche 1 bis 5 erhalten werden können, mit mindestens einer Verbindung, b) das Bestimmen der Toxizität des in Schritt a) gebildeten Komplexes an Zellen und c) das Identifizieren von mindestens einer Verbindung, die die Toxizität der Amyloid-Oligomere stört, umfasst.

7. In-vivo-Screening-Verfahren nach Anspruch 6, wobei die Zellen neuronale Zellen sind.

8. In-vitro-Screening-Verfahren zur Identifizierung von Verbindungen, die die Toxizität von Amyloid-Oligomeren stören, wobei das Verfahren a) das Bilden von Amyloid-Oligomeren wie in Anspruch 1, 2, 3 oder 4 in Gegenwart mindestens einer Verbindung, b) das Nachweisen der Hemmung der Bildung von Amyloid-Oligomeren und c) das Identifizieren von mindestens einer Verbindung, die die Toxizität der Amyloid-Oligomere stört, umfasst.

9. Verfahren nach Anspruch 8, wobei der Nachweisschritt b) spektrophotometrisch ist.

## Revendications

1. Procédé in vitro de production d'oligomères amyloïdes à partir de fibres amyloïdes comprenant la formation d'une émulsion de lipide-fibres amyloïdes en mettant en contact lesdites fibres amyloïdes avec au moins un lipide.

2. Procédé selon la revendication 1, dans lequel ledit au moins un lipide est un lipide biologique tel qu'un ganglioside, de la sphingomyéline ou du cholestérol.

3. Procédé selon la revendication 1 ou 2, comprenant en outre la centrifugation de ladite émulsion et la retenue de la fraction de surnageant.

4. Procédé selon l'une quelconque des revendications 1 à 3, dans lequel lesdites fibres amyloïdes sont constituées de la protéine bêta, tau, prion amyloïde ou d'un fragment de celle-ci.

5. Procédé selon la revendication 4, dans lequel les oligomères amyloïdes résultants sont toxiques pour les cellules neuronales.

6. Procédé de criblage in vivo chez un animal non humain pour identifier des composés qui interfèrent avec la toxicité d'oligomères amyloïdes comprenant a) la mise en contact des oligomères amyloïdes pouvant être obtenus par un procédé selon l'une quelconque des revendications 1 à 5 avec au moins un composé, b) la détermination de la toxicité du complexe formé à l'étape a) sur des cellules et c) l'identification d'au moins un composé qui interfère avec la toxicité desdits oligomères amyloïdes.

7. Procédé de criblage in vivo selon la revendication 6, dans lequel lesdites cellules sont des cellules neuronales.

8. Procédé de criblage in vivo pour identifier des composés qui interfèrent avec la formation d'oligomères amyloïdes comprenant a) la formation d'oligomères amyloïdes selon la revendication 1, 2, 3 ou 4 en présence d'au moins un composé, b) la détection de l'inhibition de la formation d'oligomères amyloïdes et c) l'identification d'au moins un composé qui interfère avec la formation d'oligomères amyloïdes.

9. Procédé selon la revendication 8, dans lequel ladite étape de détection b) est spectrophotométrique.
